# EUROPEAN PATENT SPECIFICATION

(11) **EP 0 861 246 B1**
(45) Date of publication and mention of the grant of the patent: **15.10.2003**
(21) Application number: 96925926.6
(22) Date of filing: 26.07.1996
(51) Int. Cl.: C07D 403/06, A61K 31/33, C07D 401/14, C07D 417/14, C07D 471/04

(54) **PROLYL ENDOPEPTIDASE INHIBITORS, THEIR PREPARATION AND THEIR PHARMACEUTICAL USE**
PROLYLENDOPEPTIDASE-INHIBITOREN, IHRE HERSTELLUNG UND IHRE PHARMAZEUTISCHE VERWENDUNG
INHIBITEURS DE LA PROPYLENDOPEPTIDASE, LEUR PREPARATION ET LEUR UTILISATION PHARMACEUTIQUE

(30) Priority: 17.08.1995 HU 9502426
(43) Date of publication of application: 02.09.1998
(73) Proprietor: Sandor, Erdö Dr., 2092 Budakeszi (HU)
(72) Inventor: KANAI, Károly, H-1055 Budapest (HU); ERDO, Sándor, H-1126 Budapest (HU); SZAPPANOS, Andrea, H-1101 Budapest (HU); BENCE, Judit, H-1031 Budapest (HU); HERMECZ, István, H-1056 Budapest (HU); SZVOBODA, Györgyné, H-2120 Dunakeszi (HU); BATORI, Sándor, H-1214 Budapest (HU); HEJA, Gergely, H-1131 Budapest (HU); BALOGH, Mária, H-2120 Dunakeszi (HU); HORVATH, Agnes, H-1021 Budapest (HU); SIPOS, Judit, H-1116 Budapest (HU); BARTANE, Bodor, Veronika, H-1043 Budapest (HU); PARKANY, Zsolt, H-1094 Budapest (HU); LAKICS, Viktor, H-1173 Budapest (HU); MOLNAR, Péter, H-2143 Kistarcsa (HU)
(74) Representative: Beetz & Partner Patentanwälte
(86) International application number: PCT/HU96/00041
(87) International publication number: WO 97/007116

(56) References cited:
- EP-A- 0 372 484
- EP-A- 0 419 683
- EP-A- 0 468 469
- JOURNAL OF MEDICINAL CHEMISTRY, vol. 37, no. 13, - 1994 WASHINGTON US, pages 2071-2078, XP002022213 YOSHIAKI TANAKA ET AL: "New potent prolyl endopeptidase inhibitors: ..."

## Description

The present invention relates to new compounds of the general formula (I), to pharmaceutical compositions containing them, and to a process for the preparation of these compounds. The new compounds of the general formula I are useful for the treatment of CNS diseases by inhibition of certain enzymes described later on.

Because of the incidence and social consequences of diseases of the central nervous system accompanied with amnesia, dementia and the progressive decline of cognitive and intellectual functioning, for example with Alzheimer disease, AIDS dementia, senile dementias of various origin (hypoxia, ischaemia), there are significant demands for new pharmaceuticals for treating and preventing the diseases mentioned above.

Prolyl endopeptidase PE or PEP is a post-proline cleaving enzyme (PPCE). It is widespread in mammalian species and can be found in various organs of the body. The level of the enzyme is the highest in the brain, testes and skeletal muscles (Yoshimoto, T., Ogita, K., Walter, R., Koida, M. and Tsuru, D.: Biochem. Biophys. Acta, 569, (1979), 184-192).

PEP has some important roles in the memory process due to the fact that its substrates are biologically active neuropeptides (substance P, thyrotropin-releasing hormone, Arg⁸-vasopressin). These neuropeptides exert characteristic pharmacological effects on the central nervous system: they are capable of changing the performance of animals and humans in learning and memory tasks (Toide, K., Iwamoto Z., Fujiwara, T., and Abe, H.: J. Pharm. Exp. Therapeutics, 274, (1995), 1370-1378; Riedel, W. and Jolles, J.. Drugs & Aging, 8, (1996), 245-274). The neuropeptide substance P prevents β-amyloid-induced neuronal loss and expression of Alz-50 proteins in cerebral cortex (Kowall, N., Beal, M.F., Busciglio, J., and Duffy, L.K.: Proc. Natl. Acad. Sci., 88, (1991), 7247-7251). In brains of patients with Alzheimer's disease, it is well known that the cerebral ACh content decreased, and the cerebral function suffers from severe damage (O'Leary, R. and O'Connor, B.: J. Neurochem., 65, (1995), 953-963). A PEP inhibitor, by increasing the level of TRH, could induce ACh release in the brain which should result in a better cognitive performance. It can be supposed that a highly specific PEP inhibitor could prove to be useful in the treatment of diseases of the central nervous system in neurodegenerative illnesses.

EP 0 419 683 A1 discloses prolylendopeptidase inhibitors on the basis of amino acid derivatives which comprise carbonyl-prolyl (or thioprolyl)-pyrrolidine (or thiazolidine) and groups coupled, eventually through alkylene and/or oxy or NH groups, to a 2-oxo-1-pyrrolidinyl or a phenylalkyl group.

Suitable PEP inhibitors as new drugs should be a
1. nootropic drug having memory enhancing and anti-amnestic effect and be used in treatment of age-related cognitive decline;
2. neuroprotective agent useful in the therapy of
   a. acute events (ischemia/hypoxia)
   b. progressive neurodegenerative disorders

   - Alzheimer's disease
   - AIDS dementia
   - Huntington's disease.

Senile dementia and Alzheimer's disease become serious and fastly outgrowing problem of the aging population and a PEP inhibitor could be useful for the general treatment of the above mentioned serious diseases.

It is the problem underlying the present invention to provide new PEP inhibitors showing a higher PEP inhibition level, methods for their preparation and their pharmaceutical use.

The above problem is solved according to the independent claims. The dependent claims relate to preferred embodiments of the invention.

The new PEP-inhibitors of the present invention display advantageous characteristics and may serve as active ingredients of new drugs. The advantages are a strong PEP-inhibitory effect, selectivity, easy transfer through the blood-brain barrier, a long half-life, good oral resorption, enhanced chemical and biological stability and an advantageous therapeutical profile including lower toxicity and low probability of side effects.

The compounds of the present invention are of the general formula (I), 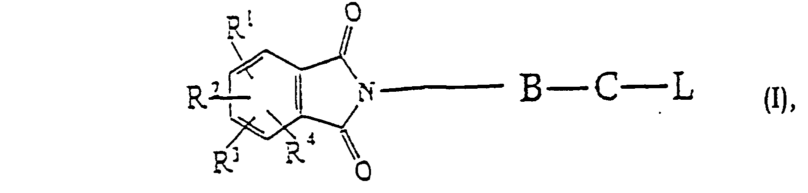 wherein are:
**R**^{**1**}**, R**^{**2**}**, R**^{**3**} **and R**^{**4**} independently
   - hydrogen,
   - halogen,
   - hydroxy,
   - cyano,
   - nitro,
   - amino,
   - anilino,
   - mercapto,
   - carboxy,
   - phenyl,
   - phenoxy,
   - benzyl,
   - benzyloxy,
   - benzoyl,
   - cyanato,
   - isocyanato,
   - thiocyanato,
   - isothiocyanato,
   - sulfamino,
   - sulfamoyl,
   - alkylthio containing 1 to 6 carbon atoms,
   - straight chain or branched chain alkyl alkenyl, alkinyl, alkoxy, alkenyloxy or alkinyloxy containing 1 to 6 carbon atoms,
   - monoalkylamino or monoacylamino containing 1 to 12 carbon atoms,
   - dialkylamino or diacylamino containing 2 to 24 carbon atoms, where the acyl group is of alkyl, aralkyl, cycloalkyl or aryl type,
   - esterified carboxy containing 2 to 7 carbon atoms,
   - hydroxyalkyl containing 1 to 6 carbon atoms,
   - acyl containing 1 to 7 carbon atoms or
   - acyloxy containing 1 to 7 carbon atoms;
**B** a group of the formula 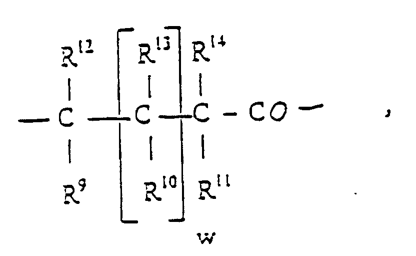
   **R**^{**9**}**, R**^{**10**}**, R**^{**11**}**, R**^{**12**}**, R**^{**13**} **and R**^{**14**} being independently
      - hydrogen,
      - phenyl,
      - phenyl substituted by 1, 2 or 3 halogen atoms or
      - alkyl containing 1 to 6 carbon atoms, and
         w being 0 or 1;
**C**
   - prolyl,
   - 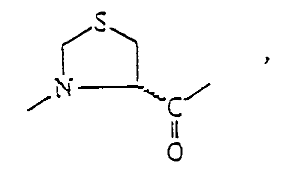
   - 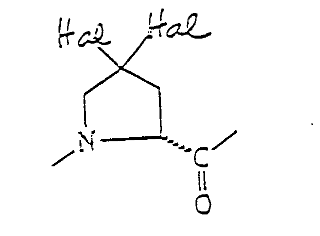 or
   - 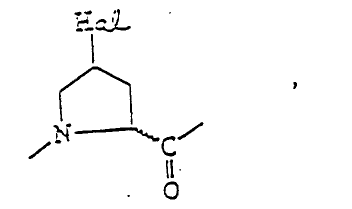 Hal being F, Cl, Br or I,
   - 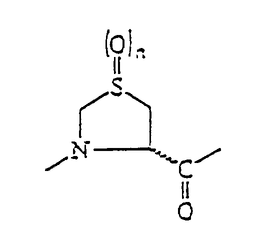 or
   - 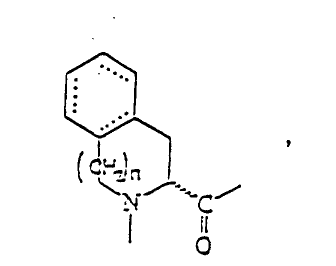 n being 0, 1 or 2,
   - 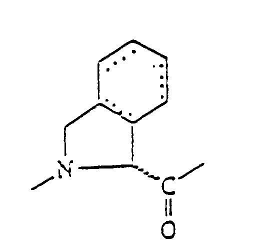 the dotted lines indicating chemical bonds optionally present, and
**L**
   - pyrrolidino,
   - 2-cyanopyrrolidino,
   - thiazolidino or
   - 2-cyanothiazolidino,
   optionally substituted with one halogen atom or geminally substituted with two halogen atoms,
   - 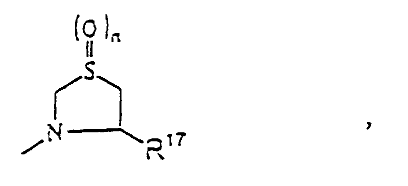 R¹⁷ being hydrogen or cyano, and
      n being 0, 1 or 2,
   - 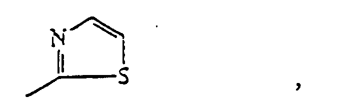
and the salts of the compounds of formula (I),
the optical isomers, cis-trans isomers, geometric isomers, epimers and tautomers being included.

Preferred compounds of the present invention are compounds of formula (I) wherein are:
**B** -(CH₂)ₘ-CO-,
   m being 2 or 3,
**C**
   - prolyl,
   - 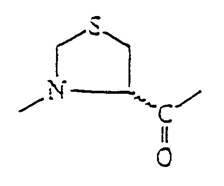 or
   - 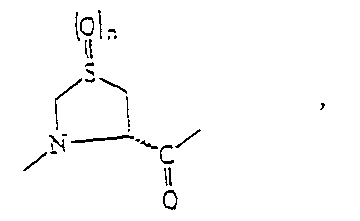 n being 1 or 2,
      and
**L**
   - pyrrolidino
      or
   - thiazolidino,
   and the salts of these compounds of formula (I),
   the optical isomers, cis-trans-isomers, geometric isomers, epimers and tautomers being included.

The process of the present invention for the preparation of the compounds of the general formula (I) as defined above is characterized by
- transforming a compound of formula (II), 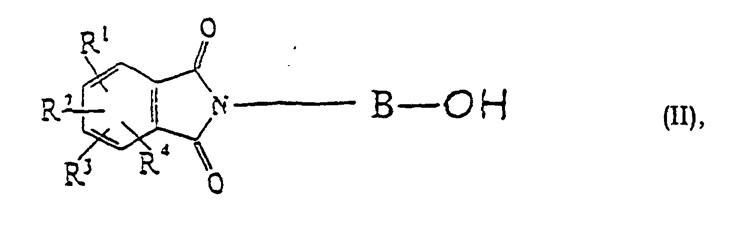 wherein R¹ to R⁴ and B are as defined above,
   into an acid halide, an active ester, a mixed acid anhydride or a carbodiimide,
   and
- allowing the resulting compound to react with a racemic or optically active compound of formula (III),

   H-C-L (III),

   wherein C and L are as defined above,
   or a salt thereof,
- and optionally separating the resulting compound of formula (I) into its optical isomers, cis-trans isomers, geometric isomers, epimers or tautomers.

According to a preferred embodiment, the compound of formula (I) is liberated from a salt thereof.

Further preferred embodiments are independently characterized by the following features:
- An acid addition salt of the compound of formula (III) is used;
- a reactive mixed anhydride of a compound of formula (II) is formed using pivaloyl chloride;
- the reaction is carried out in an organic solvent;
- the reaction is carried out at a temperature between -25 °C and the boiling point of the reaction mixture;
- the reaction is carried out in the presence of an acid binding agent.

The pharmaceutical compositions of the present invention comprise one or more of the compounds of formula (I) together with usual carriers and/or auxiliary materials.

The present invention further pertains to the use of the compounds of formula (I) as defined above for preparing pharmaceutical compositions for the inhibition of the prolylendopeptidase (PEP) enzyme in mammals including man.

In the definitions of the general formula (I), "alkyl group containing 1 to 6 carbon atoms" means a straight chain or branched alkyl group having 1 to 6 carbon atoms, such as methyl, ethyl, propyl, butyl, isobutyl, sec-butyl, tert-butyl, pentyl, neopentyl and hexyl. The "aryl group of 6 to 10 carbon atoms" means for example phenyl, tolyl or naphthyl groups.

Aralkyl groups of 6 to 10 carbon atoms are for example benzyl, 1-phenylethyl, 2-phenylethyl and 1-phenylpropyl. Alkenyl group containing 1 to 6 carbon atoms means a straight chain or branched alkenyl group such as vinyl, allyl, methacryl, crotyl, 3-butenyl, 2-pentenyl, 4-pentenyl, 2-hexenyl or 5-hexenyl. "Alkinyl containing 1 to 6 carbon atoms" means a straight-chain or branched alkinyl group such as ethinyl, propargyl, 2-butinyl, 3-butinyl, 2-pentinyl, 4-pentinyl, 2-hexinyl, 5-hexinyl or 4-methyl-2-hexinyl.

The cycloalkyl part of an "acyl group containing 1 to 12 carbon atoms" means for example cyclopropyl, cyclobutyl, cyclopentyl, cyclohexyl, cycloheptyl or cyclooctyl. These definitions may be used in case of alkoxy, alkenyloxy, alkinyloxy, aryloxy, aralkyloxy, phenylalkyloxy or alkylamino or acylamino groups.

The PEP-inhibitory activity and the biological stability of the compounds of formula (I) have been examined applying the following methods:

### PEP activity measurements on rat brain extract:

After removal of the cerebellum whole brain of male (Sprague-Dawley, 180-200 g) rats was homogenized in a double volume of 0.1 M Tris-HCl, 1 mM EDTA buffer, pH = 7.5 (PEP buffer). The homogenate was centrifuged for 30 min at 4 °C at 40000 g, and the supernatant, containing the enzyme, was collected. The pellet was resuspended in the same volume of buffer as in the first case and centrifuged again under the same conditions. The two supernatants were pooled and stored in 1 ml aliquots at -70 °C (for at least 3 months). The supernatant was thawn just before activity measurement and diluted in a 1:15 ratio with PEP buffer. The enzyme activity was measured by using the fluorometric method described by J.R. Atack et al. (Eur. J. Pharmacol., 205, (1991), 157-163). The enzyme reaction was performed at room temperature for 15 min in the presence of 62.5 µM Z-glycyl-prolyl-7-amino-4-methyl-coumarin (Bachem Biochem.) as a highly specific synthetic substrate of the PEP. The inhibitory effect of compounds was tested under the same conditions in the presence of 100 to 0.001 nM of the respective compounds. The formation of 7-amino-4-methyl-courmarin was detected spectrofluorometrically at 370 nm excitation and 440 nm emission wavelength. The 50 % inhibition concentration of the compounds (IC₅₀) was calculated by curve fitting of the % inhibition of the enzyme versus inhibitor concentration (M) using the Hill equation. The IC₅₀ values of the compounds of the general formula (I) are in the range of 100 nM to 1 pM.

### Pig brain PEP activity measurement

Purified pig brain prolyl endopeptidase was a kind gift from Lásló Polgár of the Enzymology Institute of the Hungarian Academy of Sciences. The enzyme solution was diluted in the reaction mixture 400000 times. Measurements were performed under the same conditions as in the case of the in vitro measurements on rat brain preparation. The compounds of the general formula (I) were shown to be also active on pig brain PEP activity.

### In vitro metabolism studies

The biological stability of prolyl endopeptidase inhibitors was studied in mouse, rat and human (preparation of the Central Chemistry Institute of The Hungarian Academy of Sciences) liver microsomal preparations. Mouse and rat livers were pooled and homogenized in 4-fold volume of Tris-HCl buffer (pH 7.4) containing 1.15 % KCl and 1 mM EDTA. The homogenates were centrifuged for 30 min at 10000 g, the supernatants were further ultracentrifuged for 1 h at 105000 g. The Pellets were rehomogenized, and the ultracentrifugation was repeated. The pellets were rehomogenized again and were diluted with buffer to a final volume of 0.5 g liver/ml. The samples were frozen in 2 ml aliquots at -80 °C. Preparations were characterized for cytochrome P450 isoenzyme activities.

The new inhibitors of the general formula (I) were tested under the following conditions: The reaction mixture contained 2 mg of liver microsomal protein, 0.1 M Tris-HCl buffer (pH = 7.4), 2 mM NADP, 20 mM glucose-6-phospate disodium salt, 10 mM MgCl₂, 5 U glucose-6-phosphate dehydrogenase and 50 µm PEP inhibitors in a final volume of 1.5 ml. After 0, 10, 20, 40 min incubation times, the reaction was terminated by addition of acetonitrile. The samples were centrifuged at 3000 rpm for 10 min. The supernatant was analyzed by HPLC (Supelcosil C18). The unchanged substrate amount was determined, and the half-lifes of the compounds were calculated.

Some compounds of the general formula (I) had a half-life on human liver microsomes of more than 7 h. Such a good biological stability favours a long lasting effect in vivo and is an advantage over other peptidic-type PEP-inhibitors which are known to be biologically unstable.

EP 0 232 849 A2 describes numerous PEP-inhibitors including SUAM-1221 (N-[N-(γ-phenyl)-butyryl-L-prolyl]-pyrrolidine). The compounds of the general formula (I) exert a high inhibition activity on prolyl endopeptidase which is greater than that of above reference compound SUAM-1221 measured in our above-described test system. Table 1 shows a typical example.

**Table 1**

| **Compound** | **IC**₅₀**(M) rat brain extract** |
|---|---|
| Example 23 | 3.60·10⁻¹⁰ |
| SUAM-1221 | 3.12·10⁻⁸. |

The preparation of compounds of the general formula (I) is carried out by methods well known from the literature or by obvious chemical equivalents thereof relating to the synthesis of peptide-type substances.

The phthalimido group and the B units of the compounds of the general formula (I) are coupled by reaction of the appropriate acid anhydride or other activated acid derivative and an amine, yielding compounds of the general formula (II) as described above. The coupling of the units C and L to yield compounds of the general formula (III) is carried out by reacting the appropriate mixed anhydride and the amine or an ester and a metallo-organic compound, respectively.

The starting compounds corresponding to the phthalimido unit and the units B, C and L are commercially available or easy to obtain by known transformation of them or as described in Chem. Pharm. Bulletin 41 (9) (1993), p. 1583-1588.

Compounds of the general formula (I) were prepared by reacting activated derivatives of compounds of the general formula (II) with compounds of the general formula (III) under conditions of amide coupling usual in peptide chemistry. The activated derivatives of compounds of the general formula (II) could be e.g. acid chlorides, which can be synthesized by applying halogenating agents (e.g. thionyl chloride). Active esters can be produced with 1-hydroxy-benzotriazole in the presence of N,N'-dicyclohexylcarbodiimide (Chem. Ber. 103, (1970), 788). Mixed anhydrides can be produced using esters of chloroformic acid or with pivaloyl chloride (Methoden der Organischen Chemie (Houben-Weyl), Band XV/2 Synthese von Peptiden, Georg Thieme Verlag, Stuttgart, 1974).

The coupling reaction can favourably be carried out in an organic solvent, preferably at a temperature between -25 °C and the boiling point of the reaction mixture. The use of acid binding agents, e.g. organic amines, is favourable during the reaction.

The compounds of the general formula (I), can be purified, if appropriate, by a conventional purification technique, the isomers of which are separated, if desired, by a conventional separation technique and which are converted, if necessary, to their addition salts with a pharmaceutically acceptable acid.

Pharmaceutically acceptable acids may be for example hydrochloric, sulfuric, tartaric, fumaric or methanesulfonic acid and the like.

Another subject-matter of the present invention are pharmaceutical compositions containing, as active ingredient, at least one compound of the general formula (I) or one of its addition salts with a pharmacologically acceptable acid, alone or in combination with one or more inert and non-toxic excipients or vehicles.

Mention may more particularly be made, among pharmaceutical compositions according to the invention, of those which are suitable for oral, parenteral, rectal or nasal administration, and which may be simple or sugar-coated tablets, sublingual tablets, injectable compositions, infusions, packets, gelatine capsules, suppositories, creams, ointments, dermal gels, and the like.

The dose varies according to the age and weight of the patient, the nature and the severity of the ailment and the administration route.

The latter can be oral, nasal, rectal or parenteral. The unit dose generally varies between 0.1 and 50 mg/kg body weight for a treatment taken with 1 to 3 administrations per 24 h.

The invention will be further explained by the following non-limiting examples in greater detail, whereby a detailed process description is given for the compound of example 2. Other embodiments of the invention will be apparent to the person skilled in the art from a consideration of this specification or the practice of the invention disclosed herein.

### Examples

### Description of the preparation of compound depicted in Example 2 (Table 2)

To a solution prepared by dissolving 1.17 g (5.0 mmol) 4-phthalimidobutyric acid and 0.56 g (5.5 mmol) triethylamine in 20 ml chloroform 0.61 g (5.0 mmol) pivaloyl chloride were dropped at -15 °C under stirring. The reaction mixture was stirred for 1 h at the above temperature and then a solution prepared by dissolving 1.03 g (5,0 mmol) L-prolylpyrrolidin-hydrochloric acid salt in a mixture of 5 ml chloroform and 1.5 ml (1.1 g, 11.0 mmol) triethylamine were dropwise added thereto. The reaction mixture was stirred at room temperature for 4 h, then it was washed successively with water, 30 % cc. citric acid solution, saturated aqueous sodium bicarbonate solution, water and with saturated sodium chloride solution. The organic phase was dried on calcinated magnesium sulfate and was then evaporated. Crystallisation of the residue from a mixture of 5 ml chloroform and 10 ml petrolether yielded 1.1 g (53 %) N-(4-phthalimido-butanoyl)-L-prolylpyrrolidin which melted at 148-149 °C. The compounds of the general formula (I) were synthetized by the method as explained above starting from the corresponding compounds having the general formulae (II) and (III).

The structures and the physical constants of several novel compounds of the general formula (I) are listed in Table 2.

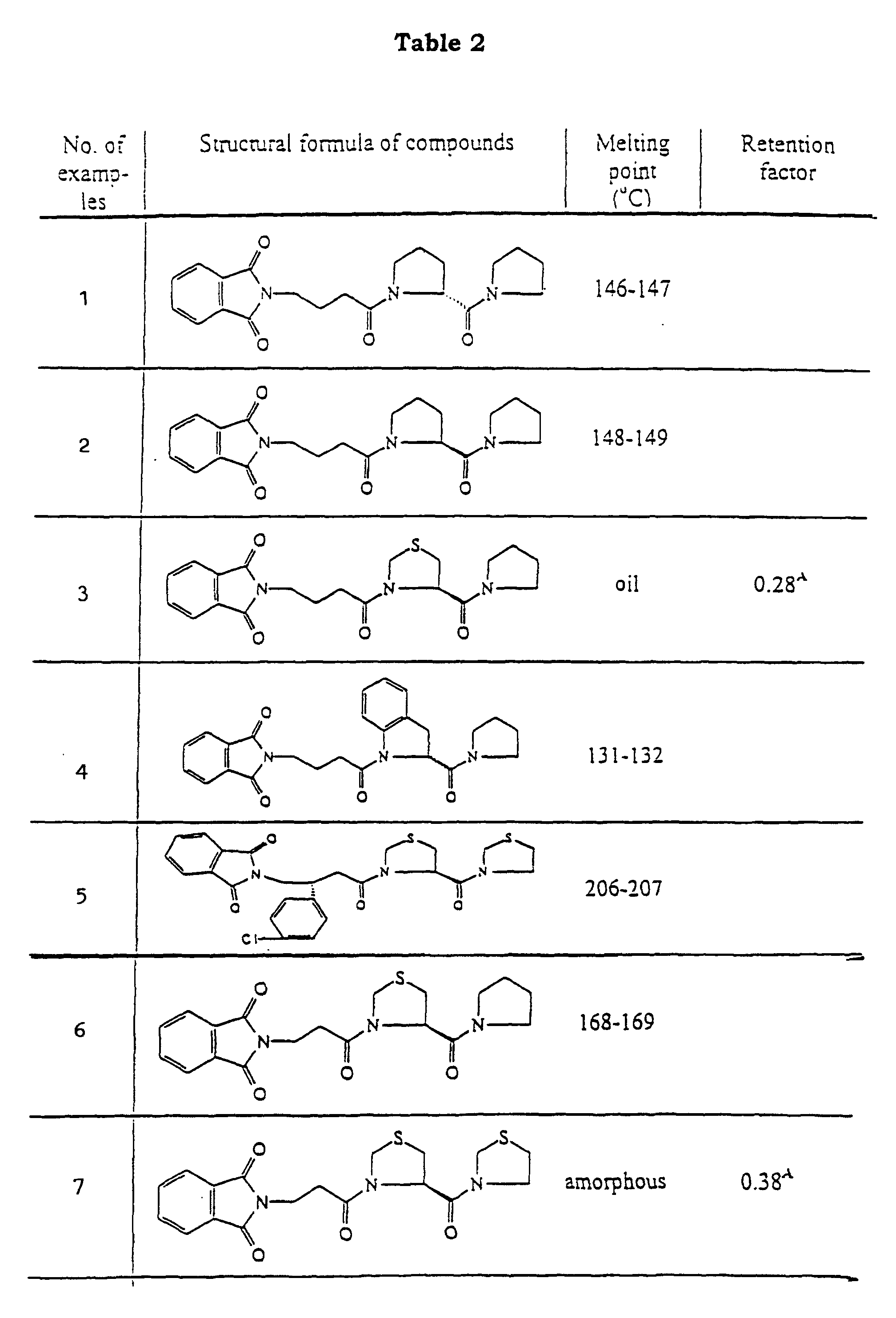

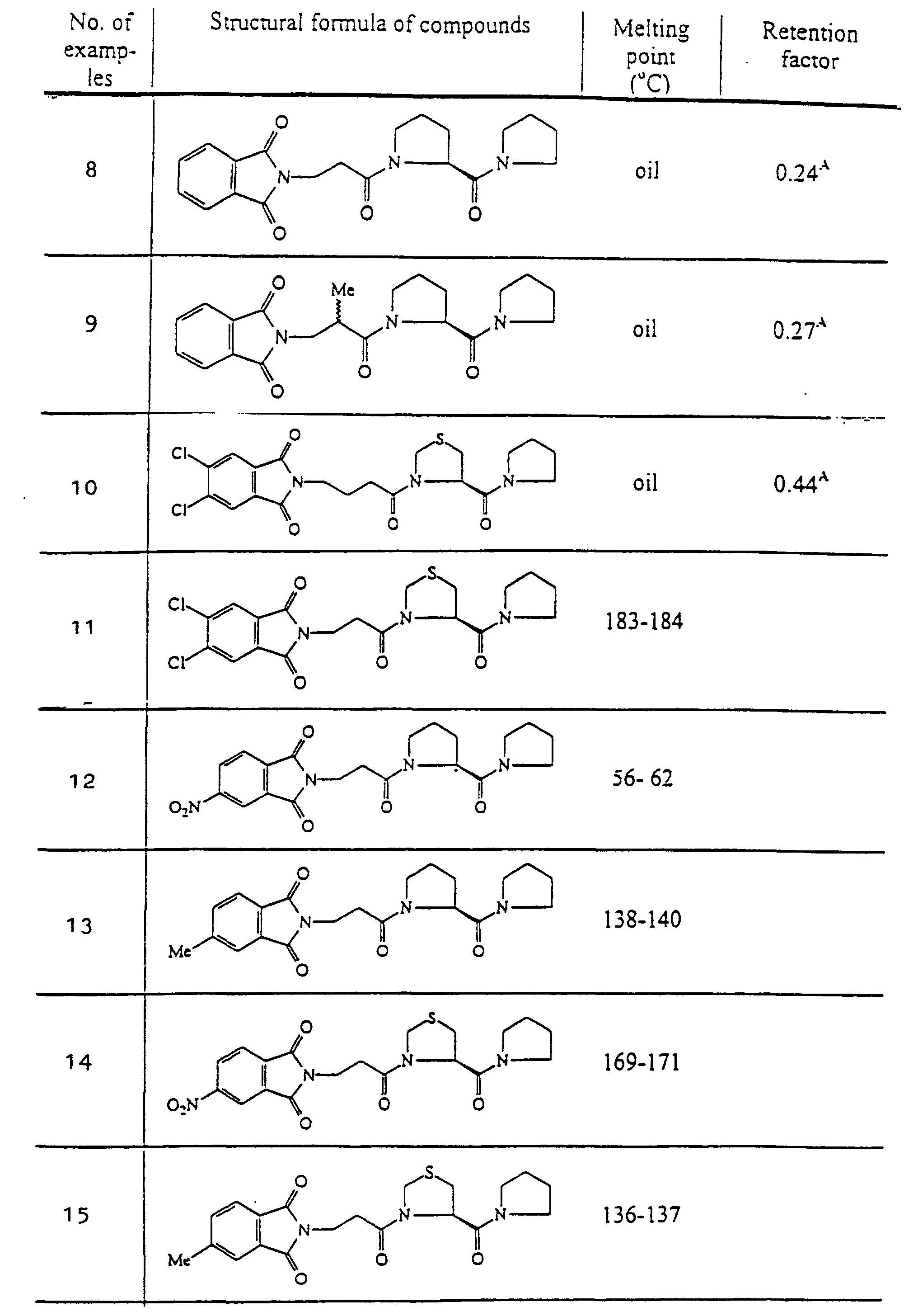

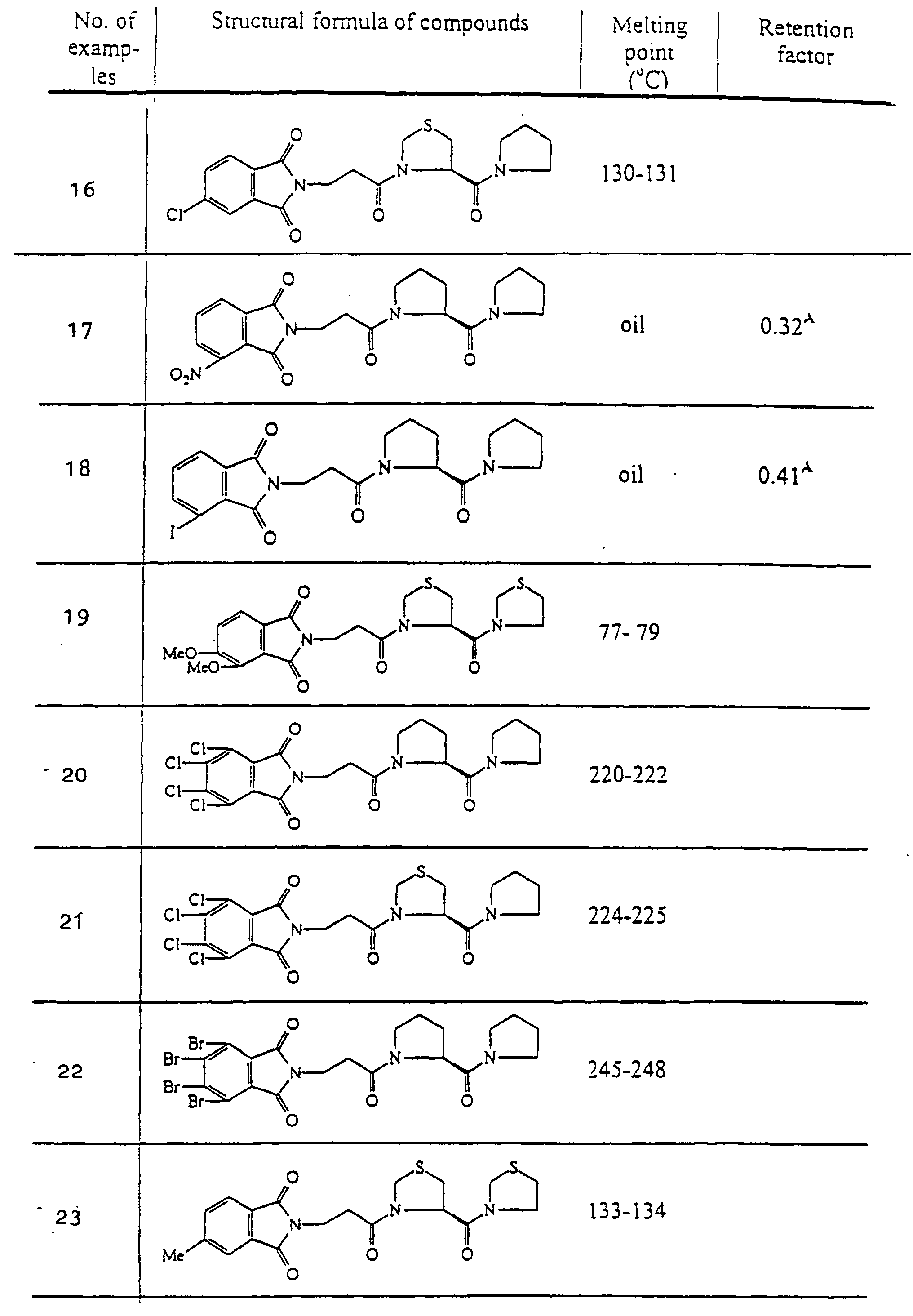

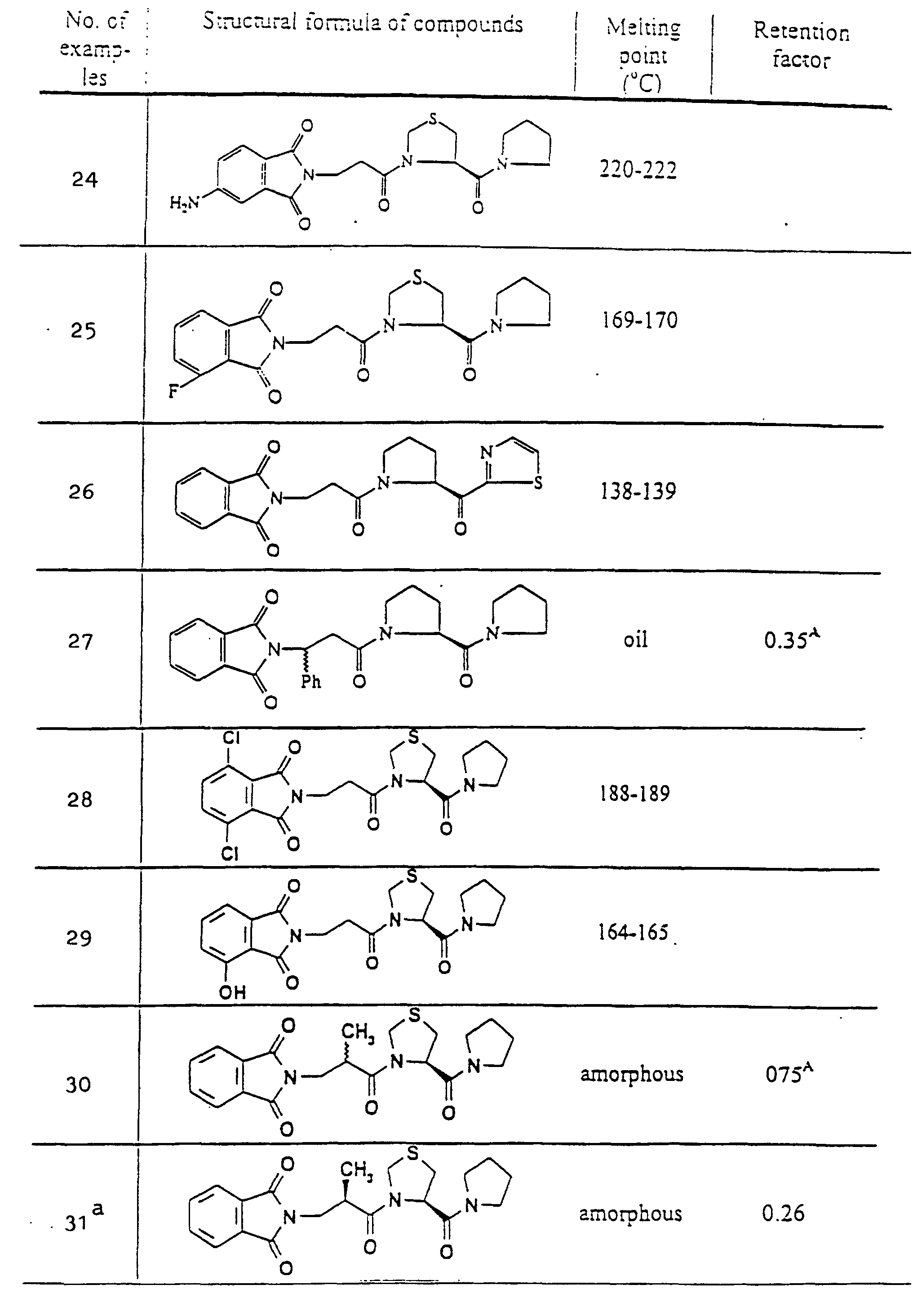

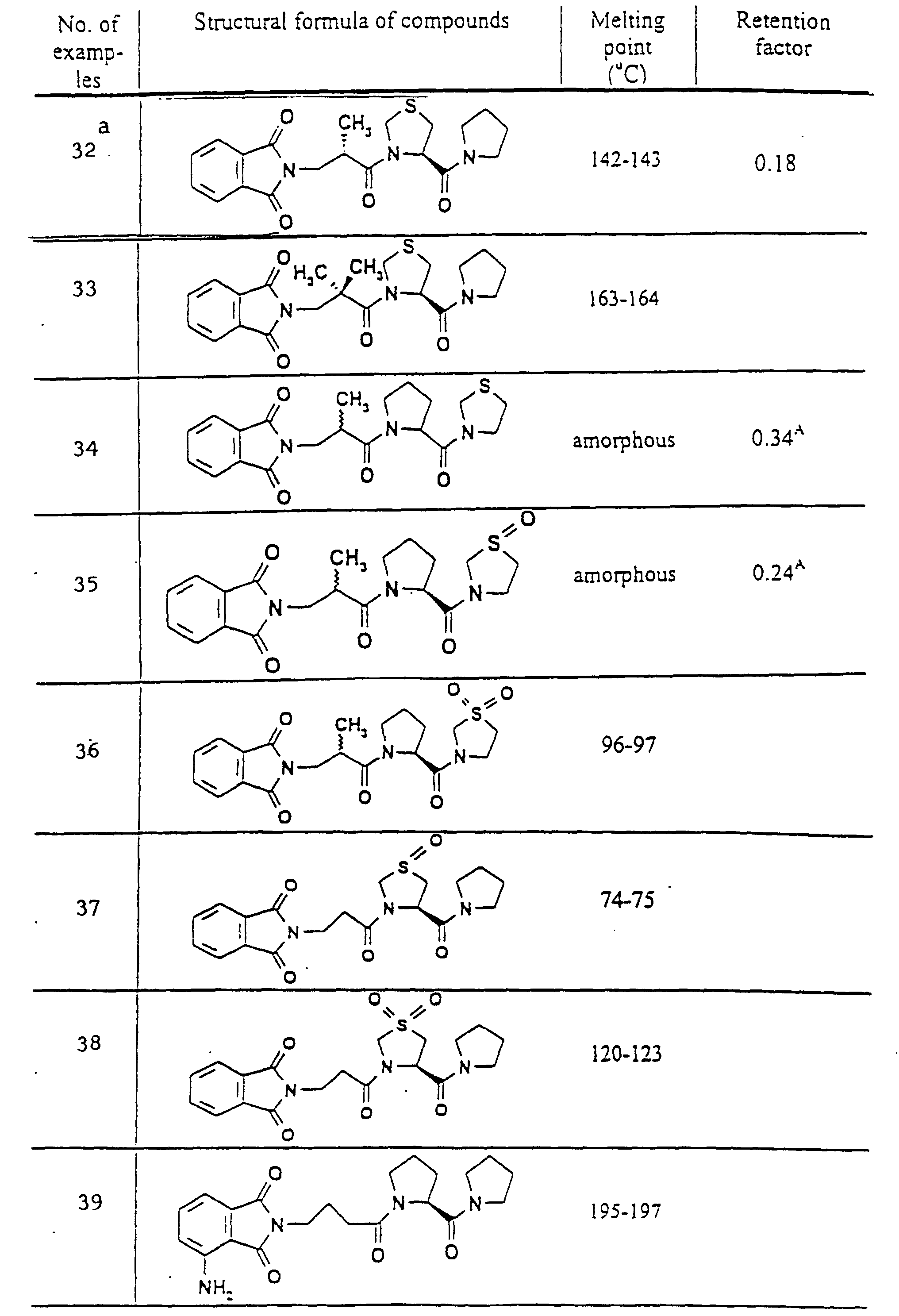

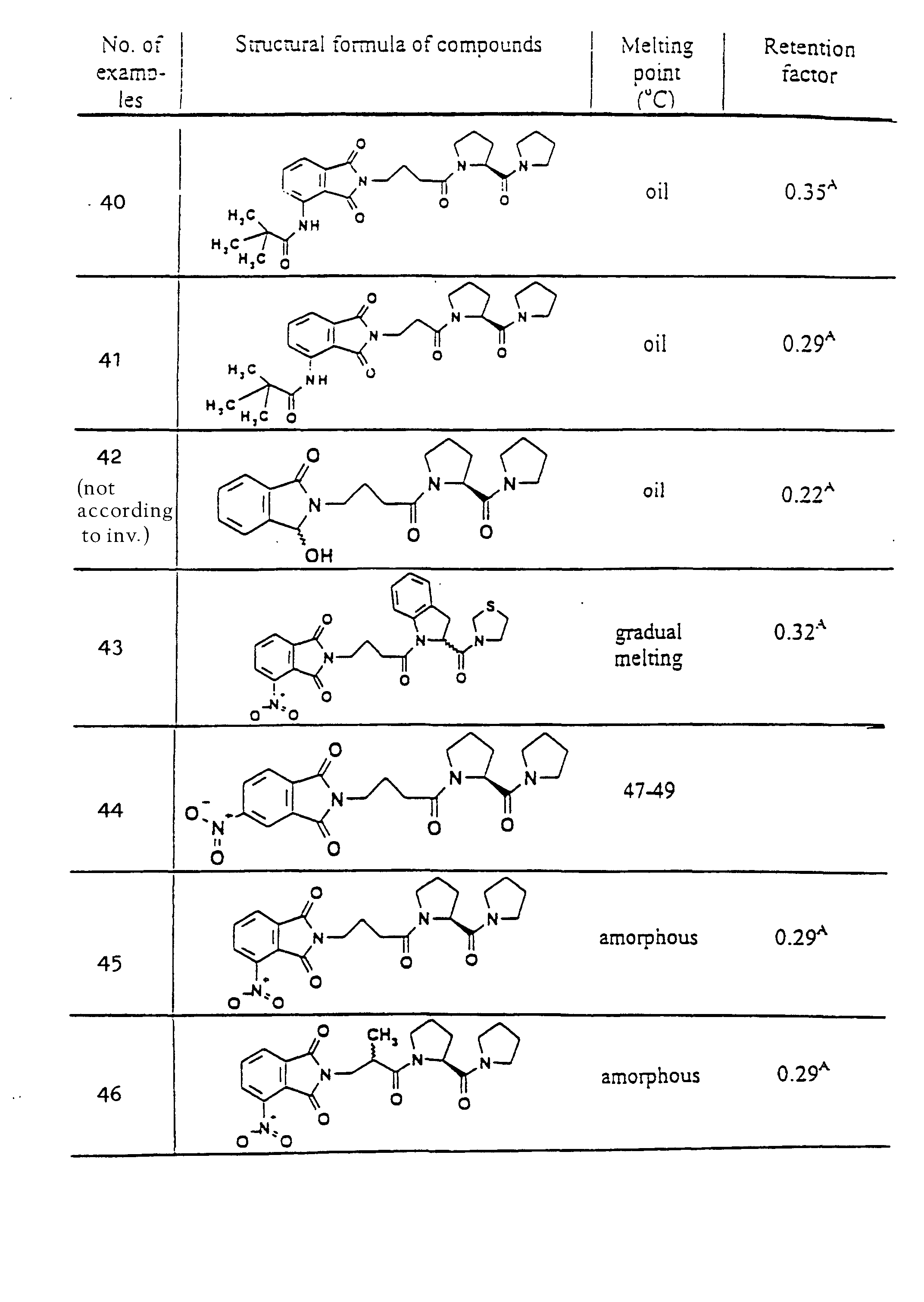

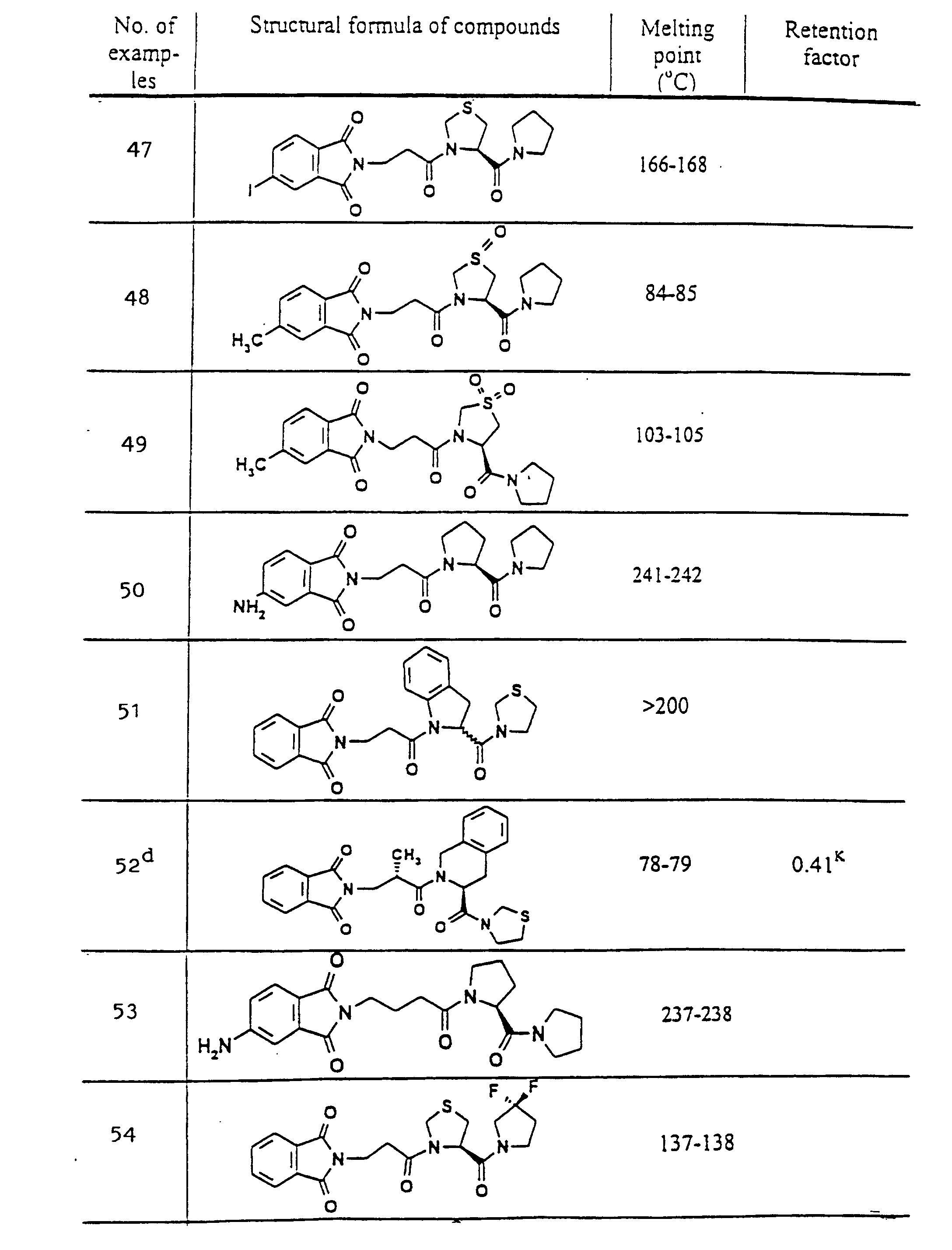

a, d
tentative assignment of epimers, may be reverse

### Abbreviations of eluents:

A Chloroform:methanol = 20:1
K Chloroform:acetone = 10:1

## Claims

1. Compounds of the general formula (I), 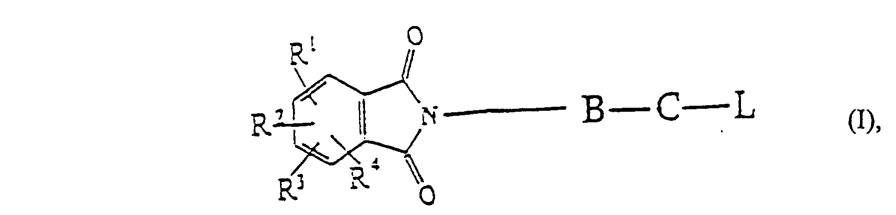 wherein are:
**R**^{**1**}**, R**^{**2**}**, R**^{**3**} **and R**^{**4**} independently
• hydrogen,
• halogen,
• hydroxy,
• cyano,
• nitro,
• amino,
• anilino,
• mercapto,
• carboxy,
• phenyl,
• phenoxy,
• benzyl,
• benzyloxy,
• benzoyl,
• cyanato,
• isocyanato,
• thiocyanato,
• isothiocyanato,
• sulfamino,
• sulfamoyl,
• alkylthio containing 1 to 6 carbon atoms,
• straight chain or branched chain alkyl alkenyl, alkinyl, alkoxy, alkenyloxy or alkinyloxy containing 1 to 6 carbon atoms,
• monoalkylamino or monoacylamino containing 1 to 12 carbon atoms,
• dialkylamino or diacylamino containing 2 to 24 carbon atoms, where the acyl group is of alkyl, aralkyl, cycloalkyl or aryl type,
• esterified carboxy containing 2 to 7 carbon atoms,
• hydroxyalkyl containing 1 to 6 carbon atoms,
• acyl containing 1 to 7 carbon atoms
or
• acyloxy containing 1 to 7 carbon atoms;
**B** a group of the formula 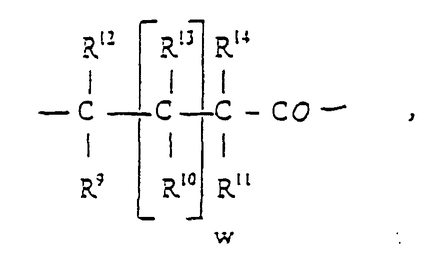
**R**^{**9**}**, R**^{**10**}**, R**^{**11**}**, R**^{**12**}**, R**^{**13**} **and R**^{**14**} being independently
- hydrogen,
- phenyl,
- phenyl substituted by 1, 2 or 3 halogen atoms
or
- alkyl containing 1 to 6 carbon atoms,
and
w being 0 or 1;
**C**
• prolyl,
• 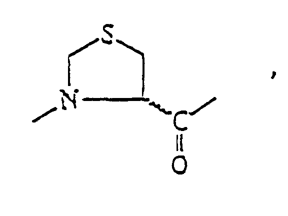
• 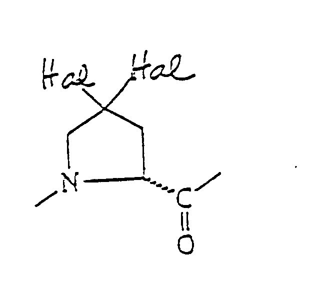 or
• 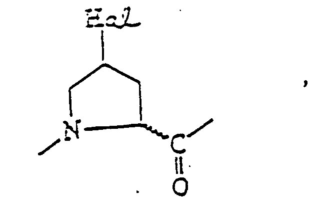 Hal being F, Cl, Br or I,
• 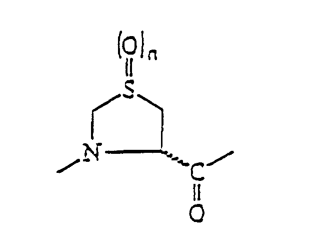 or
• 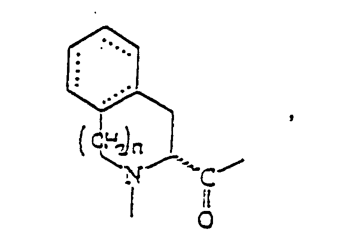 n being 0, 1 or 2,
• 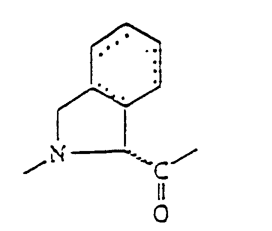 the dotted lines indicating chemical bonds optionally present,
and
**L**
• pyrrolidino,
• 2-cyanopyrrolidino,
• thiazolidino or
• 2-cyanothiazolidino,
optionally substituted with one halogen atom or geminally substituted with two halogen atoms,
• 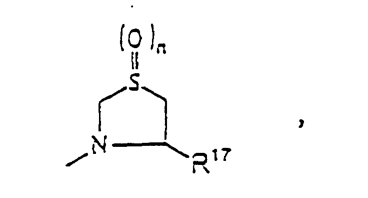 R¹⁷ being hydrogen or cyano, and
n being 0, 1 or 2,
• 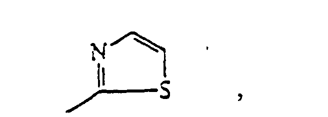
and the salts of the compounds of formula (I),
the optical isomers, cis-trans isomers, geometric isomers, epimers and tautomers being included.

2. Compounds of formula (I) according to claim 1,
wherein are:
**B** -(CH₂)ₘ-CO-,
m being 2 or 3,
**C**
• prolyl,
• 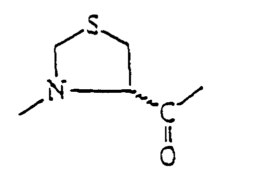 or
• 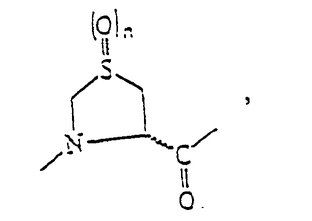 n being 1 or 2, and
**L**
• pyrrolidino
or
• thiazolidino,
and the salts of these compounds of formula (I),
the optical isomers, cis-trans-isomers, geometric isomers, epimers and tautomers being included.

3. A process for the preparation of compounds of the general formula (I) as defined in claim 1 or 2,
**characterized by**
- transforming a compound of formula (II), 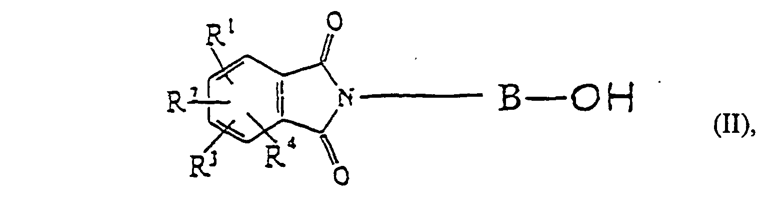 wherein R¹ to R⁴ and B are as defined in claim 1 or 2,
into an acid halide, an active ester, a mixed acid anhydride or a carbodiimide, and
- allowing the resulting compound to react with a racemic or optically active compound of formula (III),
H-C-L (III),
wherein C and L are defined as in claim 1 or 2,
or a salt thereof,
- and optionally separating the resulting compound of formula (I) into its optical isomers, cis-trans isomers, geometric isomers, epimers or tautomers.

4. A process for the preparation of compounds of the general formula (I) as defined in claim 1 or 2, **characterized by** liberation of the compound of formula (I) from a salt thereof.

5. A process according to claim 3, **characterized in that** an acid addition salt of the compound of formula (III) is used.

6. A process according to claim 3 or 5, **characterized in that** a reactive mixed anhydride of a compound of formula (II) is formed using pivaloyl chloride.

7. A process according to any of claims 3, 5 or 6, **characterized in that** the reaction is carried out in an organic solvent.

8. A process according to any of claims 3, 5, 6 or 7, **characterized in that** the reaction is carried out at a temperature between -25 °C and the boiling point of the reaction mixture.

9. A process according to any of claims 3, 5, 6, 7 or 8, **characterized in that** the reaction is carried out in the presence of an acid binding agent.

10. Pharmaceutical compositions, comprising one or more of the compounds of formula (I) according to claim 1 or 2, together with usual carriers and/or auxiliary materials.

11. Use of the compounds of formula (I) as defined in claim 1 or 2 for preparing pharmaceutical compositions for the inhibition of the prolylendopeptidase (PEP) enzyme in mammals including man.

## Patentansprüche

1. Verbindungen der allgemeinen Formel (I), 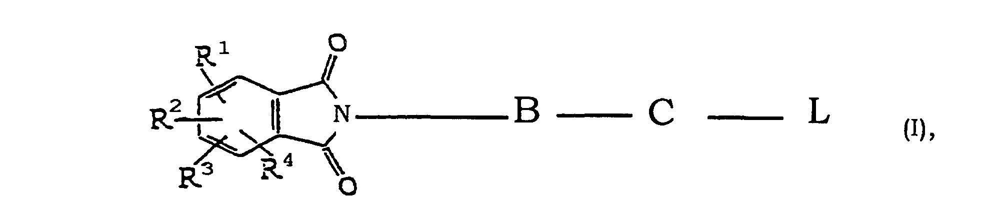 worin bedeuten:
R¹, R², R³ und R⁴ unabhängig
• Wasserstoff,
• Halogen,
• Hydroxy,
• Cyano,
• Nitro,
• Amino,
• Anilino,
• Mercapto,
• Carboxy,
• Phenyl,
• Phenoxy,
• Benzyl,
• Benzyloxy,
• Benzoyl,
• Cyanato,
• Isocyanato,
• Thiocyanato,
• Isothiocyanato,
• Sulfamino,
• Sulfamoyl,
• Alkylthio mit 1 bis 6 Kohlenstoffatomen,
• geradkettiges oder verzweigtes Alkyl, Alkenyl, Alkinyl, Alkoxy, Alkenyloxy oder Alkinyloxy mit 1 bis 6 Kohlenstoffatomen,
• Monoalkylamino oder Monoacylamino mit 1 bis 12 Kohlenstoffatomen,
• Dialkylamino oder Diacylamino mit 2 bis 24 Kohlenstoffatomen, wobei die Acylgruppe vom Alkyl-, Aralkyl-, Cycloalkyl- oder Aryltyp ist,
• verestertes Carboxy mit 2 bis 7 Kohlenstoffatomen,
• Hydroxyalkyl mit 1 bis 6 Kohlenstoffatomen,
• Acyl mit 1 bis 7 Kohlenstoffatomen
oder
• Acyloxy mit 1 bis 7 Kohlenstoffatomen;
**B** eine Gruppe der Formel 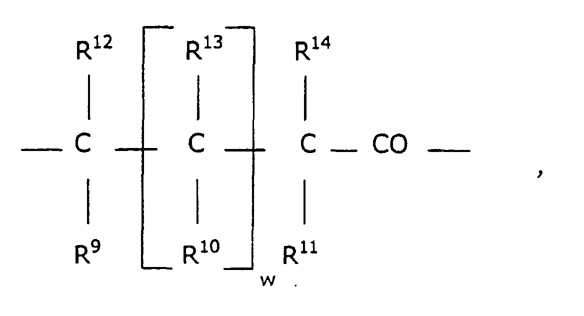
in der R⁹, R¹⁰, R¹¹, R¹², R¹³ und R¹⁴ unabhängig bedeuten:
- Wasserstoff,
- Phenyl,
- mit 1, 2 oder 3 Halogenatomen substituiertes Phenyl oder
- Alkyl mit 1 bis 6 Kohlenstoffatomen,
wobei
w gleich 0 oder 1 ist;
**C**
• Prolyl,
• 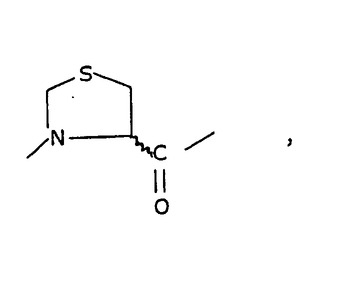
• 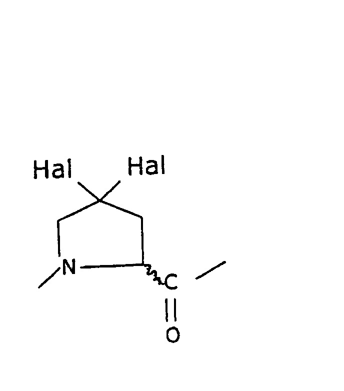 oder
• 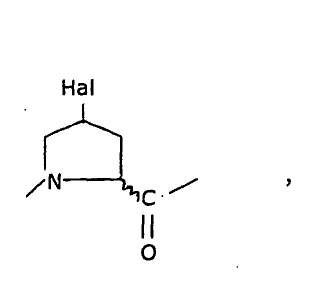
wobei Hal F, Cl, Br oder I bedeutet,
• 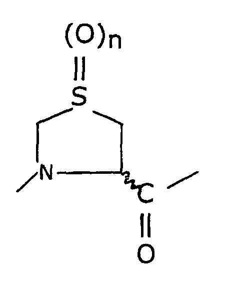 oder
• 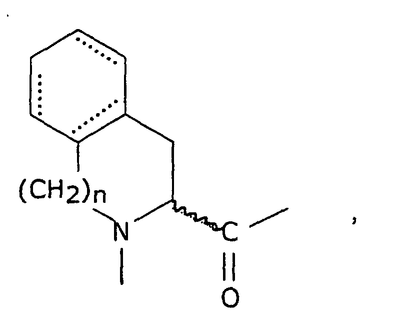 wobei n 0, 1 oder 2 bedeutet,
• 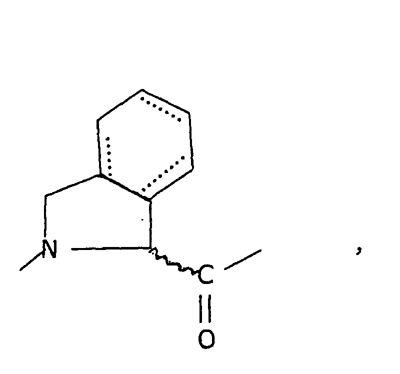 wobei die gestrichelten Linien gegebenenfalls vorliegende chemische Bindungen bedeuten,
und
**L**
• Pyrrolidino,
• 2-Cyanopyrrolidino,
• Thiazolidino oder
• 2-Cyanothiazolidino,
die gegebenenfalls mit einem Halogenatom oder geminal mit zwei Halogenatomen substituiert sind,
• 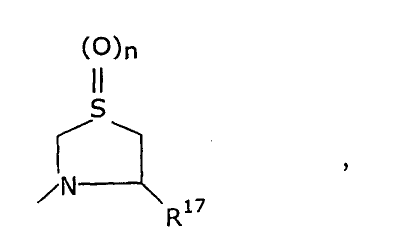 wobei R¹⁷ Wasserstoff oder Cyano und
n 0, 1 oder 2 bedeuten,
• 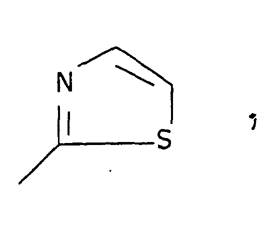
und die Salze der Verbindungen der Formel (I),
wobei die optischen Isomeren, die cis-trans-Isomeren, die geometrischen Isomeren, die Epimeren und die Tautomeren eingeschlossen sind.

2. Verbindungen der Formel (I) nach Anspruch 1, worin bedeuten:
**B** -(CH₂)ₘ-CO-,
wobei m 2 oder 3 bedeutet,
**C**
• Prolyl,
• 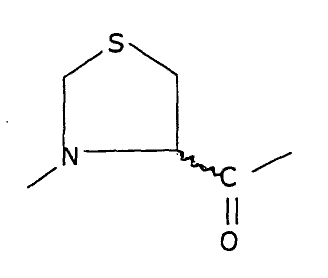 oder
• 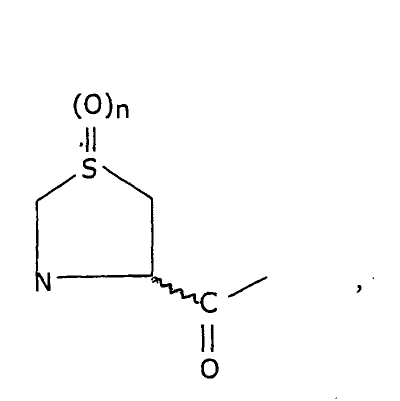
wobei n 1 oder 2 bedeutet,
und
**L**
• Pyrrolidino
oder
• Thiazolidino,
sowie die Salze dieser Verbindungen der Formel (I),
wobei die optischen Isomeren, die cis-trans-Isomeren, die geometrischen Isomeren, die Epimeren und die Tautomeren eingeschlossen sind.

3. Verfahren zur Herstellung von Verbindungen der allgemeinen Formel (I) wie in Anspruch 1 oder 2 definiert,
**gekennzeichnet durch**
- Umwandlung einer Verbindung der Formel (II), 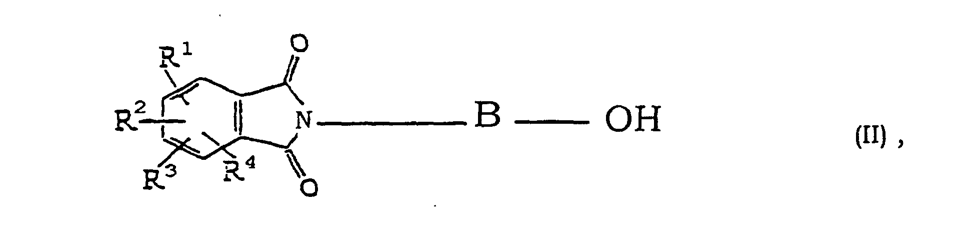 in der R¹ bis R⁴ und B wie in Anspruch 1 oder 2 definiert sind,
in ein Säurehalogenid, einen aktiven Ester, ein gemischtes Säureanhydrid oder ein Carbodiimid
und
- Umsetzung der resultierenden Verbindung mit einer racemischen oder optisch aktiven Verbindung der Formel (III),
H-C-L (III)
worin C und L wie in Anspruch 1 oder 2 definiert sind,
oder einem Salz davon,
- und wahlweise Auftrennung der resultierenden Verbindung der Formel (I) in ihre optischen Isomeren, cis-trans-Isomeren, geometrischen Isomeren, Epimeren oder Tautomeren.

4. Verfahren zur Herstellung von Verbindungen der allgemeinen Formel (I) wie in Anspruch 1 oder 2 definiert, **gekennzeichnet durch** Freisetzung der Verbindung der Formel (I) aus einem ihrer Salze.

5. Verfahren nach Anspruch 3, **dadurch gekennzeichnet, dass** ein Säureadditionssalz der Verbindung der Formel (III) eingesetzt wird.

6. Verfahren nach Anspruch 3 oder 5, **dadurch gekennzeichnet, dass** ein reaktives gemischtes Anhydrid einer Verbindung der Formel (II) unter Verwendung von Pivaloylchlorid hergestellt wird.

7. Verfahren nach einem oder mehreren der Ansprüche 3, 5 oder 6, **dadurch gekennzeichnet, dass** die Umsetzung in einem organischen Lösungsmittel durchgeführt wird.

8. Verfahren nach einem oder mehreren der Ansprüche 3, 5, 6 oder 7, **dadurch gekennzeichnet, dass** die Umsetzung bei einer Temperatur zwischen -25 °C und dem Siedepunkt des Reaktionsgemisches durchgeführt wird.

9. Verfahren nach einem oder mehreren der Ansprüche 3, 5, 6, 7 oder 8, **dadurch gekennzeichnet, dass** die Umsetzung in Gegenwart eines säurebindenden Mittels durchgeführt wird.

10. Pharmazeutische Zusammensetzungen, die eine oder mehrere Verbindungen der Formel (I) nach Anspruch 1 oder 2 zusammen mit üblichen Trägern und/oder Hilfsstoffen enthalten.

11. Verwendung der Verbindungen der Formel (I) wie in Anspruch 1 oder 2 definiert zur Herstellung von pharmazeutischen Zusammensetzungen zur Inhibierung des Enzyms Prolylendopeptidase (PEP) bei Säugern einschließlich des Menschen.

## Revendications

1. Composés de formule générale (I), 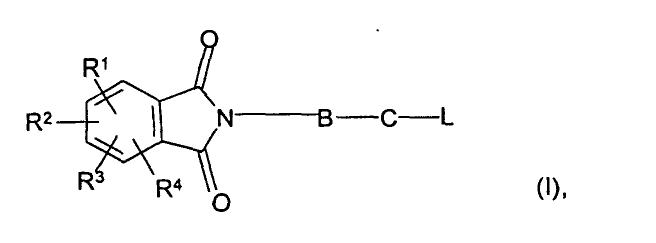 dans laquelle
**R**^{**1**}**, R**^{**2**}**, R**^{**3**} **et R**^{**4**} sont, indépendamment les uns des autres :
• hydrogène,
• halogène,
• hydroxy,
• cyano,
• nitro,
• amino,
• anilino,
• mercapto,
• carboxy,
• phényle,
• phénoxy,
• benzyle,
• benzyloxy,
• benzoyle,
• cyanato,
• isocyanato,
• thiocyanato,
• isothiocyanato,
• sulfamino,
• sulfamoyl,
• alkylthio comprenant de 1 à 6 atomes de carbone,
• alkyle, alkényle, alkinyle, alkoxy, alkényloxy ou alkinyloxy à chaîne linéaire ou ramifiée comprenant de 1 à 6 atomes de carbone,
• monoalkylamino ou monoacylamino comprenant de 1 à 12 atomes de carbone,
• dialkylamino ou diacylamino comprenant de 2 à 24 atomes de carbone, où le groupe acyle est du type alkyle, aralkyle, cycloalkyle ou aryle,
• carboxy estérifié comprenant de 2 à 7 atomes de carbone,
• hydroxyalkyle comprenant de 1 à 6 atomes de carbone,
• acyle comprenant de 1 à 7 atomes de carbone
ou
• acyloxy comprenant de 1 à 7 atomes de carbone;
**B** est un groupe de formule : 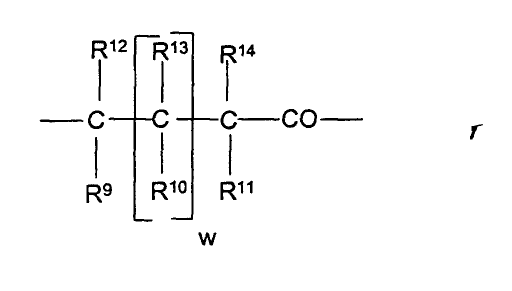 dans laquelle
**R**⁹, **R**¹⁰, **R**¹¹, **R**¹², **R**¹³ et **R**¹⁴ sont, indépendamment les uns des autres :
- hydrogène,
- phényle,
- phényle substitué par 1, 2 ou 3 atomes d'halogène
ou
- alkyle comprenant de 1 à 6 atomes de carbone,
et
w est égal à 0 ou 1;
**C** représente :
• prolyle,
• 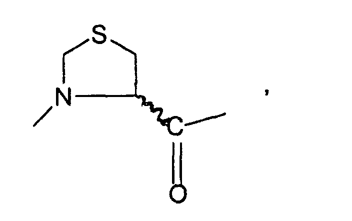
• 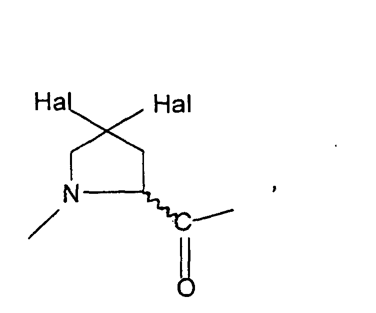 ou
• 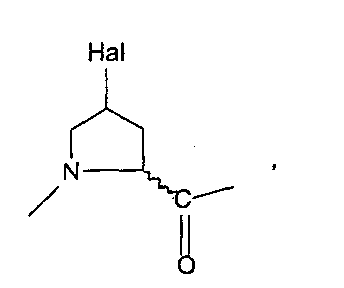 Hal représentant F, Cl, Br ou I,
• 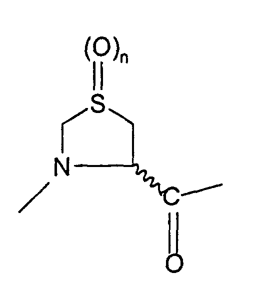 ou
• 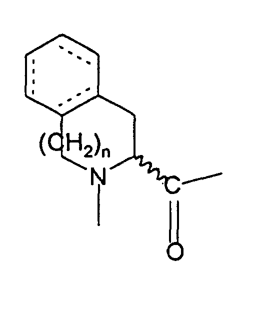 n étant égal à 0, 1 ou 2, 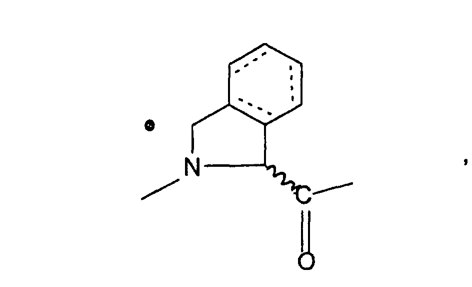
les lignes en pointillés indiquant des liaisons chimiques éventuellement présentes,
et
**L** représente
• pyrrolidino,
• 2-cyanopyrrolidino,
• thiazolidino ou
• 2-cyanothiazolidino,
éventuellement substitués par un atome d'halogène ou substitués par deux atomes d'halogène géminaux,
• 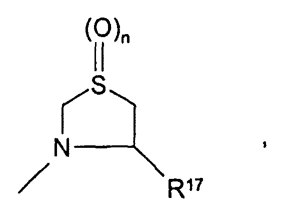 R¹⁷ étant hydrogène ou cyano, et
n étant égal à 0, 1 ou 2,
• 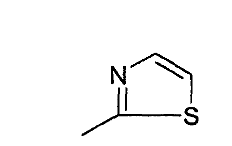
et les sels des composés de formule (I),
les isomères optiques, isomères cis-trans, isomères géométriques, les épimères et les tautomères étant compris.

2. Composés de formule (I) selon la revendication 1,
dans lequel
**B** représente un groupe -(CH₂)ₘ-CO-,
m étant égal à 2 ou 3,
**C** représente
• prolyle,
• 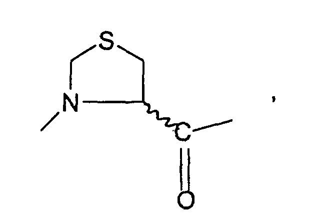 ou
• 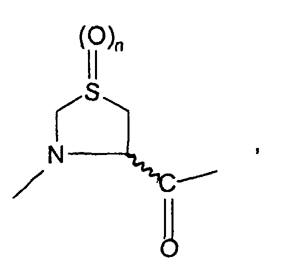 n étant égal à 1 ou 2,
et
**L** représente
• pyrrolidino,
ou
• thiazolidino,
et les sels de ces composés de formule (I),
les isomères optiques, isomères cis-trans, isomères géométriques, les épimères et les tautomères étant compris.

3. Procédé pour la préparation de composés de formule générale (I) selon la revendication 1 ou 2,
**caractérisé par**
- la transformation d'un composé de formule (II), 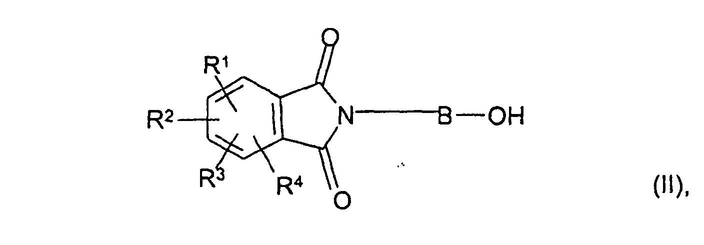 dans laquelle R¹ à R⁴ et B sont tels que définis dans la revendication 1 ou 2,
en un halogénure d'un acide, un ester actif, un anhydride acide mixte ou un carbodiimide
et
- permettant la réaction du composé résultant avec un composé racémique ou optiquement actif de formule (III),
H-C-L (III),
dans laquelle C et L sont tels que définis dans la revendication 1 ou 2, ou un sel de celui-ci,
- et éventuellement la séparation du composé résultant de formule (I) en ses isomères optiques, isomères cis-trans, isomères géométriques, les épimères ou les tautomères.

4. Procédé pour la préparation de composés de formule générale (I) selon la revendication 1 ou 2, **caractérisé par** la libération du composé de formule (I) d'un de ses sels.

5. Procédé selon la revendication 3, **caractérisé en ce qu'**un sel d'addition du composé de formule (III) avec un acide est utilisé.

6. Procédé selon la revendication 3 ou 5, **caractérisé en ce qu'**un anhydride réactif mixte d'un composé de formule (II) est formé en utilisant le chlorure de pivaloyle.

7. Procédé selon l'une quelconque des revendications 3, 5 ou 6, **caractérisé en ce que** la réaction est réalisée dans un solvant organique.

8. Procédé selon l'une quelconque des revendications 3, 5, 6 ou 7, **caractérisé en ce que** la réaction est réalisée à une température comprise entre -25°C et le point d'ébullition du mélange de réaction.

9. Procédé selon l'une quelconque des revendications 3, 5, 6, 7 ou 8, **caractérisé en ce que** la réaction est réalisée en présence d'un accepteur d'acide.

10. Compositions pharmaceutiques, comprenant un ou plusieurs des composés de formule (I) selon la revendication 1 ou 2, en compagnie des excipients et/ou auxiliaires usuels.

11. Utilisation des composés de formule (I) selon la revendication 1 ou 2 pour la préparation de compositions pharmaceutiques pour l'inhibition de l'enzyme prolylendopeptidase (PEP) chez les mammifères, y compris l'être humain.
